# EUROPEAN PATENT APPLICATION

(11) **EP 0 812 906 A1**
(43) Date of publication of application: **17.12.1997**
(21) Application number: 96870073.2
(22) Date of filing: 10.06.1996
(51) Int. Cl.: C11D 3/39, D06L 3/02, A61K 7/20, A61K 7/30, A61K 7/135, A61K 7/06

(54) **Liquid bleaching compositions with hydroperoxides**

(71) Applicant: THE PROCTER & GAMBLE COMPANY, Cincinnati, Ohio 45202 (US)
(72) Inventor: Del Duca, Valerio, 80063 Massa Lubrense (NA) (IT); Scialla, Stefano, 00128 Roma (IT); Bianchetti, Giulia Ottavia, 00144 Roma (IT)
(74) Representative: Engisch, Gautier

(57) **Abstract**

Liquid compositions are described which are acidic and which comprise an alkyl hydroperoxide or an organomineral hydroperoxide.

The compositions do not cause skin itching, unlike conventional peroxygen, and are useful for bleaching fabrics, carpets, toilets, dentures, teeth and hair.

## Description

### Technical Field

The present invention relates to liquid bleaching compositions comprising a peroxygen bleach. The compositions are useful for a variety of applications. The compositions are formulated so as to avoid itching of the skin of the user when it comes in contact with the composition.

### Background

Bleaching with peroxygen bleaches is well-known in the art. Peroxygen bleaching finds a variety of applications, such as bleaching fabrics or, in the household, treating hard surfaces such as toilets, or softer surfaces such as carpets. In such applications, accidental contact of the composition and the user's skin may occur. Peroxygen beaches are also useful in health applications such as the bleaching of hair, of denture, of teeth, most of which require contact with the user's skin. A major drawback of liquid peroxygen bleaches is that they may bleach the skin of the person using the composition and whiten it. This whitening effect is fully reversible, but it also causes temporary itching of the skin, which causes discomfort to the user.

A possible compromise to address this problem is to use rather low levels of the peroxygen bleach, but that is of course detrimental to performance.

It is thus an object of the present invention to provide a liquid peroxygen bleach composition which is formulated so as to avoid this temporary itching phenomenon.

It has now been found that hydroperoxides are peroxygen bleaching agents which cause less or no skin itching, as compared to conventional hydrogen peroxide bleaches, especially when formulated in an acidic or neutral composition.

### Summary of the Invention

The present invention encompasses a liquid composition comprising a peroxygen bleach, which is formulated at an acidic or neutral pH and wherein the peroxygen bleach is an alkyl or organomineral hydroperoxide.

The compositions of the present invention are useful in the bleaching of fabrics, carpets, toilets, dentures, teeth and hair. Accordingly the present invention also encompasses processes wherein these items are contacted with the composition comprising the hydroperoxide.

### Detailed Description of the Invention

The compositions herein comprise a hydroperoxide. Suitable alkyl hydroperoxides for use herein include alkyl hydroperoxides and organomineral hydroperoxides.

Suitable alkyl hydroperoxides for use herein are according to the formula 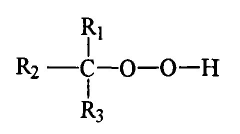 in which each R1, R2 and R3 is, independently, a hydrogen atom or a hydrocarbon radical having from 1 to 30 carbon atoms. The hydrocarbon radical can be a linear or a cyclic hydrocarbon chain, and the linear and the cyclic hydrocarbon chain can be straight or branched, saturated or unsaturated. Also, two of the R groups can be part of the same cyclic hydrocarbon. One or more R groups in the above formula can be a single or condensed aromatic radical, alkyl-aryl radical or cycloalkyl-aryl radical.

All the above R radicals can also be substituted by heteroatoms or group of heteroatoms, such as hydroperoxide (-OOH) groups, halogen atoms, hydroxy groups, nitrates, suphonyls, nitro groups, ethers, carboxylic groups and esters. All these groups can also be present as substituents of one or more positions of the R radicals.

Preferred alkyl hydroperoxides for use herein are tert-butyl hydroperoxide, cumyl hydroperoxide, 2,4,4-trimethylpentyl-2-hydroperoxide, diisopropylbenzene-monohydroperoxide, tert-amyl hydroperoxide and 2,5-dimethyl-hexane-2,5-dihydroperoxide.

Suitable organomineral hydroperoxides for use herein are according to the formula

RₘM(OOH)ₙ

where M is a metal atom like Si, Sn, Ge or Sb and R is a radical defined as R1, R2, R3 above. Preferred organomineral hydroperoxides for use herein are (CH₃)₃SiOOH, (C₆H₅)₂CH₃SiOOH, (C₆H₅)₃SiOOH, (n-C₆H₁₃)₃SiOOH, (CH₃)₃SnOOH, (C₆H₅)₃Sb(OOH)₂. Most preferred for use herein are (CH₃)₃SiOOH, (C₆H₅)₃SiOOH.

The compositions herein comprise from 0.01% to 5.0%, preferably 0.01% to 4.5%, most preferably 0.02% to 4.0% of available oxygen provided by said hydroperoxide.

The compositions to be used according to the present invention must be liquids. As used herein, "liquid" includes "pasty" and "gel" compositions, and liquid compositions herein preferably have a viscosity of from 5 cps to 20000 cps at 50 rpm shear rate and at 20°c temperature.

The compositions herein are acidic or neutral. Preferred pH is from 2 to 7, and most preferred pH is of from 5 to 6.5. Indeed, we have found that optimum hydroperoxide stability is obtained in that range. The pH of the compositions can be trimmed by conventional acidifying agents, such as sulphuric acid.

Depending on the end-use envisioned, the composition herein can contain a variety of additional ingredients, such as surfactants, solvents, builders or chelants, antioxidants, and additives such as perfume and dyes.

### Examples

### Example 1

A liquid composition is prepared as follows:

| | |
|---|---|
| Cumyl Hydroperoxide | 10% |
| Dobanol 23.3 ® | 8.6% |
| Dobanol 45.7 ® | 6.4% |
| C12 Alkyl Sulfate | 2% |
| Water and minors | up to 100% |
| pH = 4, trimmed with Sulphuric acid | |

This composition is useful as a laundry bleach. It is directly applied on the stained portion of a fabric and left to act threon for 5 minutes. Then the fabric is washed.

### Example 2

The following liquid composition is prepared:

| | |
|---|---|
| tert-Butyl Hydroperoxide | 10% |
| Dobanol 45.7 ® | 6.0% |
| Dobanol 23.6,5 ® | 6.0% |
| C25-AE2.5-S (ethoxylated alkyl sulfate) | 6.0% |
| Water and minors | up to 100% |
| pH=4, trimmed with Sulphuric acid | |

This composition is useful as liquid laundry additive. It is poured in the main wash together with a conventional detergent. This composition can also be used as in example 1.

### Example 3

The following liquid composition is prepared:

| | |
|---|---|
| tert-Butyl Hydroperoxide | 5% |
| Dobanol 91.10 ® | 1.6% |
| Dobanol 23.3 ® | 1.5% |
| C10 AS | 1.7% |
| Isofol 12 ® | 0.5% |
| Water and minors | up to 100% |
| pH=4, trimmed with Sulphuric acid | |

This composition is useful as a laundry bleach and can be used as described in examples 1 and 2. This composition can also be used as a soaking product, when it is dliuted in water (100 ml composition in 10 liters wate), and fabrics are contacted with this bath, and left to soak therein for a period of time of 24 hours. Fabrics are eventually rinsed.

### Example 4

The following liquid composition is prepared:

| | |
|---|---|
| tert-Butyl Hydroperoxide | 7% |
| Ukanil ® | 1.0% |
| C12 AS | 1.0% |
| Water and minors (BHT, perfume) | up to 100% |
| pH=6, trimmed with Sulphuric acid | |

This composition is useful as carpet cleaner. It is poured neat on stained carpets and left to dry. The residues formed are then removed with a vacuum cleaner machine.

### Example 5

The following liquid composition is prepared:

| | |
|---|---|
| Cumyl Hydroperoxide | 5% |
| C10 amino oxide | 1.0% |
| C16 quaternary ammonium | 2.0% |
| Water and minors (BHT, perfume) | up to 100% |
| pH=3, trimmed with Sulphuric acid | |

This composition is poured neat on the internal surface of a toilet bowl and left to act thereon before it is flushed away.

### Example 6

The following liquid composition is prepared:

| | |
|---|---|
| Tert-butyl Hydroperoxide | 15% |
| Cethyl alcohol | 2.5% |
| Arlacel 165 | 2.5% |
| Water and minors | up to 100% |
| pH=5, trimmed with phosphoric acid. | |

This composition is useful for the bleaching of hair. The pH has to be trimmed to an alkaline value just before use.

This and other hair bleaching compositions containing alkyl hydroperoxides can be prepared according to the following references:
a) The chemistry and manufacture of cosmetics, vol. 4, 2nd edition, Maison G. de Navarre (editor), chapter 45 by G.S. Kass
b) The science of hair care, edited by C. Zviak, chapter 7
c) Chemical and physical behaviour of human hair, 3rd edition by Clarence Robbins

### Example 7

The following paste composition is prepared:

| | |
|---|---|
| Tert-butyl Hydroperoxide | 5% |
| Sodium bicarbonate | 20% |
| Hydrated silica | 15% |
| Glycerin | 15% |
| Sorbitol | 14% |
| Alkyl Sulphate | 5% |
| Water and minors | up to 100% |
| pH=6.5, trimmed with pyrophosphoric acid. | |

This composition is useful as toothpaste (viscosity 5000 cps) for the bleaching of teeth. By addition of low levels of gelling agents, this composition can also be achieved as a transparent gel with viscosity of 15000 cps.

### Example 8

The following liquid composition is prepared:

| | |
|---|---|
| Tert-butyl Hydroperoxide | 5% |
| Glycerin | 5% |
| Sorbitol | 14% |
| Alkyl Sulphate | 5% |
| Disinfecting agent | 1% |
| Water and minors | up to 100% |
| pH=6.5, trimmed with ortophosphoric acid. | |

This composition is useful as liquid denture cleaner to be diluted upon usage.

## Claims

1. A liquid composition comprising a peroxygen bleach, characterized in that said peroxygen bleach is a hydroperoxide according to the formula 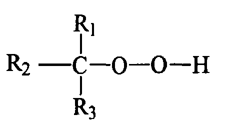 or
RₘM(OOH)ₙ
where each R₁, R₂, R₃, R is independently H or a hydrocarbon radical comprising 1 to 30 carbon atoms, and said composition is neutral or acidic.

2. A composition according to claim 1 wherein said hydroperoxide is tert-butyl hydroperoxide or cumyl hydroperoxide or 2,5-dimethyl-hexane-2,5-dihydroperoxide or 2,4,4-trimethylpentyl-2-hydroperoxide or diisopropylbenzene-monohydroperoxide or tert-amyl hydroperoxide.

3. A composition according to the preceding claims which comprises from 0.02% to 4.0% of available oxygen provided by said hydroperoxide.

4. A composition according to the preceding claims which has a pH of from 5 to 6.5

5. A process wherein a composition according to any of the preceding claims is contacted with a fabric, or a carpet, or a toilet, or a denture, or a tooth, or a hair, and said fabric or carpet or toilet or denture or tooth or hair is rinsed.
